# FASCICULE DE BREVET EUROPEEN

(11) **EP 2 099 422 B1**
(45) Date de publication et mention de la délivrance du brevet: **23.09.2015**
(21) Numéro de dépôt: 07871937.4
(22) Date de dépôt: 14.12.2007
(51) Int. Cl.: A61K 8/36, A61Q 7/00, A61K 8/97

(54) **UTILISATION ORALE D'ACIDE PÉTROSÉLINIQUE POUR AMELIORER LA CHEVELURE**
ORALE VERWENDUNG VON PETROSELINSÄURE ZUR STÄRKUNG DER HAARE
ORAL USE OF PETROSELINIC ACID FOR HAIR IMPROVEMENT

(30) Priorité: 14.12.2006 EP 06291928; 13.02.2007 US 900990 P
(43) Date de publication de la demande: 16.09.2009
(73) Titulaire: L'Oréal, 75008 Paris (FR); Nestec S.A., 1800 Vevey (CH)
(72) Inventeur: GUENICHE, Audrey, F-92500 Rueil Malmaison (FR); CASTIEL, Isabelle, F-06200 Nice (FR)
(74) Mandataire: Tanty, François
(86) Numéro de dépôt international: PCT/FR2007/052516
(87) Numéro de publication internationale: WO 2008/081142

(56) Documents cités:
- EP-A- 0 116 439
- EP-A- 0 888 773
- EP-A1- 1 013 178
- EP-A2- 0 709 084
- WO-A-01/08651
- DE-A1- 10 035 735
- anonymous: "Alopecia areata", , 15 October 2012 (2012-10-15), Retrieved from the Internet: URL:http://en.wikipedia.org/wiki/Alopecia_ areata [retrieved on 2012-10-15]
- DATABASE GNPD [Online] MINTEL; September 2001 (2001-09), Anonymous: "Vitamin Supplement", retrieved from www.gnpd.com Database accession no. 112817
- ACKMAN ET AL: "Characteristics of the fatty acid composition and biochemistry of some fresh-water fish oils and lipids in comparison with marine oils and lipids", COMPARATIVE BIOCHEMISTRY AND PHYSIOLOGY, PERGAMON, vol. 22, no. 3, 1 September 1967 (1967-09-01), pages 907-922, XP025202320, ISSN: 0010-406X, DOI: 10.1016/0010-406X(67)90781-5 [retrieved on 1967-09-01]
- RAMADAN M F ET AL: "Oil composition of coriander (Coriandrum sativum L.) fruit-seeds", EUROPEAN FOOD RESEARCH AND TECHNOLOGY, SPRINGER VERLAG, HEIDELBERG, DE, vol. 215, no. 3, 1 January 2002 (2002-01-01), pages 204-209, XP003005185, ISSN: 1438-2377, DOI: 10.1007/S00217-002-0537-7

## Description

La présente invention se rapporte à l'utilisation par administration orale d'au moins un acide gras mono-insaturé spécifique dans une composition renfermant un milieu cosmétiquement ou pharmaceutiquement acceptable, en tant qu'agent protecteur (traitement ou prévention) du cuir chevelu et des cheveux.

Le follicule pileux est formé de compartiments parfaitement individualisés, les uns d'origine dermique (gaine conjonctive et papille dermique), les autres de nature épithéliale (gaine épithéliale externe, gaine interne, tige pilaire et glande sébacée). La gaine conjonctive, synthétisée par des fibroblastes, est surtout une matrice extracellulaire formée de collagènes de type I et III, ainsi que de protéoglycannes. Traversée, dans le tiers inférieur, par un réseau de capillaires sanguins, elle se prolonge à la base du follicule, par la papille dermique, véritable agrégat de matrice extracellulaire. La papille dermique est constituée par des fibroblastes particuliers responsables de la synthèse locale de prostaglandine E2. Une membrane basale, composée de collagène de type IV, de laminine de type 1 et 5, de fibronectine et d'héparane sulfate protéoglycanes, sépare le compartiment dermique du compartiment épithélial. Le profil d'expression de la laminine 5 est toutefois interrompu à la périphérie du bulbe, soulignant ainsi une régionalisation de la jonction dermo-épidermique. Le compartiment épithélial peut, quant à lui, être séparé en quatre domaines distincts. A la base du follicule et entourant la papille dermique où se trouve la matrice, siège d'une intense activité mitotique. Cette matrice produit, avec des programmes de différenciation spécifiques, les trois grands domaines concentriques que sont la gaine externe, la gaine interne et la tige pilaire. Dans la partie supérieure du follicule se trouve la glande sébacée, formée de kératinocytes particuliers, les sébocytes responsables de la synthèse de sébum. La partie supérieure de la gaine externe est également caractérisée par la présence de cellules de Langerhans, véritables sentinelles du système immunitaire, et de cellules de Merkel remplies de neuropeptides et dont le rôle est encore méconnu (Revue dans « La vie révélée du follicule de cheveu humain »; Bernard BA, Medecine Sciences Vol 22(2), 2006).

La Demanderesse a réalisé depuis plusieurs années de nombreux tests cliniques et elle a pu ainsi déterminer les symptômes liés aux cheveux âgés. Ces symptômes sont en particulier un changement d'aspect de la fibre (cheveu fin, terne, sans tenu (mou), sans éclat, grisonnant), des cheveux qui ont tendance à chuter, qui se renouvellent moins vite. Ces manifestations résultent d'une désorganisation du réseau de collagène dans le derme du scalp, de l'apparition de produits de glycation (1) qui rigidifie le derme autour du follicule pileux entraînant des effets négatifs pour les follicules résidents et l'implantation de nouveaux cheveux, et de la réduction de la synthèse de sébum augmentant d'autant l'aspect terne et sans éclat du cheveu et favorisant la chute des cheveux (2, 3).
(1) Monnier VM, Cerami A. ACS Symposium Series 215 (1983) : 431-449
(2) Jaworsky C, Kligman AM, Murphy GF. Br J Dermatol 127 (1992) : 239-246
(3) Sueki H, Stoudemayer T et al. Acta Dermatol Venereol 79 (1999) : 347-350

Avoir des cheveux sains, vigoureux et nombreux tout au long de la vie est une ambition de la plupart des hommes et des femmes. De nombreux produits pour le traitement des cheveux sont disponibles. Cependant, aucune composition pour la voie orale ne permet à ce jour de lutter contre les altérations du cuir chevelu et/ou des cheveux apparaissant avec l'âge et ne permet de protéger le « capital cheveu ».

La Demanderesse a mis en évidence que l'acide pétrosélinique pouvai permettre de lutter contre les altérations des cheveux vieillis.

Le vieillissement chronologique est un phénomène naturel qui se manifeste progressivement et plus ou moins tôt selon les individus. Les manifestations du vieillissement sur la chevelure peuvent toutefois être accélérés par des facteurs endogènes, stress, maladies..., ou exogènes tels que la pollution, l'exposition aux UV...

Les acides gras mono-insaturés ont été décrits pour différentes applications telles que l'hydratation de la peau sèche (EP 0 709 084 de L'Oréal).

EP 0 116 439 (Suntory) divulgue que certains acides gras ont une forte activité d'inhibition de la 5α-réductase de la flore bactérienne du cuir chevelu, et peuvent être utilisés de ce fait dans les produits toniques pour le traitement des pellicules et des démangeaisons du cuir chevelu. L'action de ces acides gras vise donc la flore bactérienne du cuir chevelu.

US 4,097,604 (Oxford Hill) indique qu'un sel de différents acides gras est actif contre les micro-organismes pathogènes de la cavité buccale, permettant ainsi de diminuer l'incidence de ces bactéries sur d'apparition de periodontoses. L'action de ces acides gras est dans ce cas limitée à la flore buccale.

EP 0 355 842 (Sansho Seiyaku) décrit une crème de soin destinée à prévenir la pigmentation causée par une surproduction de mélanine, ladite crème pouvant renfermer de l'acide pétrosélinique.

EP 1 013 178 (Unilever) décrit l'utilisation d'acide pétrosélinique comme agent anti-inflammatoire et pour traiter les signes cutanés du vieillissement (ride, peau flasque, taches de sénescence).

EP 888 773 (NESTLE) décrit une composition contenant de l'acide pétrosélinique, utile pour le traitement ou la prévention des inflammations et/ou la modulation des lipides dans les tissus superficiels. Toutefois, l'utilisation cosmétique de l'acide pétrosélinique pour lutter contre les altérations du cuir chevelu et/ou des cheveux apparaissant avec l'âge n'y est ni dérite, ni suggérée.

Par ailleurs, aucun de ces documents ne laissait présager de l'activité de l'acide pétrosélinique sur la qualité de la fibre capillaire et, ainsi de la chevelure.

Ainsi, la présente invention se rapporte à l'utilisation, notamment l'utilisation cosmétique, par voie orale d'au moins un acide gras mono-insaturé, un de ses sels ou un de ses esters, pour prévenir et/ou traiter les altérations du cuir chevelu (scalp) et/ou des cheveux apparaissant avec l'âge, l'acide gras mono-insaturé étant l'acide pétrosélinique.

Les altérations du cuir chevelu et/ou des cheveux apparaissant avec l'âge s'entendent d'altérations liées au processus de vieillissement, à savoir d'altérations non médiées par un processus inflammatoire. En particulier, lesdites altérations s'entendent d'altérations uniquement liées au processus de vieillissement, qu'il soit de type chronovieillissement ou photovieillissement.

Au titre de ce processus inflammatoire non considéré dans la présente invention, on peut notamment citer celui résultant d'une infection, d'une allergie, d'une maladie auto-immune, d'une exposition à des agents irritants.

Ainsi, la présente invention concerne l'utilisation, notamment l'utilisation cosmétique, par voie orale d' acide pétrosélinique, pour prévenir et/ou traiter les altérations du cuir chevelu et/ou des cheveux apparaissant avec l'âge et non médiées par une processus inflammatoire, en particulier non médiées par un processus inflammatoire induit par une exposition à un agent irritant.

Il est entendu dans le cadre de la présente invention que « l'utilisation cosmétique par voie orale » recouvre l'utilisation de produits administrés par voie orale, ces produits étant, par exemple sous forme de complément alimentaire ou d'aliment fonctionnel, produisant un effet au niveau du cuir chevelu et/ou des cheveux sur le plan esthétique et du confort, ou encore à visée beauté, par exemple en vue de la protection, du maintien en bon état, de la modifications de l'aspect, et notamment de l'embellissement.

Les altérations du cuir chevelu, en particulier les altérations du cuir chevelu, apparaissant avec l'âge sont notamment les fibroses des tissus entourant les follicules pileux.

La Demanderesse a en effet pu mettre en évidence que les acides gras mono-insaturé conduisaient à :
- une restructucturation du réseau collagénique notamment visible par coloration au Trichorme Masson sur une coupe de peau d'un individu supplémenté avec de l'huile de coriandre, en effet, cette huile a comme activité la diminution de l'activité collagénase et élastase (mesuré par dosage enzymatique) ;
- une diminution des protéines glyquées retrouvées dans le derme et l'épiderme (diminution des dérivés carboxylmethyl-lysine) ;
- une diminution des marqueurs de stress et d'inflammation (tels que Hsp 70 et TNF-alpha dont la mesure peut être réalisée avec des anti-corps spécifiques) or le stress oxydatif du tissu environnant des follicules pileux conduit à une altération des cheveux, voire de leur chute.

Ils préviennent également l'altération du derme par les enzymes tissulaires telles que les collagènases, élastases et gelatinases et conduisent ainsi à un profil cutané, en particulier du cuir chevelu, sain, sans fibrose, favorable à la pousse d'un cheveu de bonne qualité.

L'utilisation d'acide pétrosélinique, un de ses sels ou un de ses esters permet également de limiter la chute des cheveux et/ou augmenter la densité de la chevelure.

La densité d'une chevelure se détermine en fonction du nombre de cheveux pour une surface de scalp donnée.

Selon un mode de réalisation, l'utilisation objet de l'invention est destinée à lutter contre les altérations des cheveux vieillis. Elle est également destinée à protéger les cheveux vieillis.

En particulier, l'utilisation selon l'invention permet de prévenir et/ou limiter la formation de cheveux fins et/ou ternes et/ou cassants et/ou mous.

L'utilisation selon l'invention permet également d'améliorer la qualité des fibres kératiniques, notamment en favorisant la pousse de cheveux brillants et/ou épais et/ou vigoureux.

On entend par acide gras mono-insaturé, un acide gras comprenant une unique double liaison.

Il s'agit plus particulièrement d'acides gras à longues chaînes. Les acides gras mono-insaturés convenant à l'invention sont les acides gras mono-insaturés comportant 12 à 22 atomes de carbone.

Les acides gras mono-insaturés peuvent être sous forme acide, ou de sel ou encore sous forme de dérivés notamment d'esters ou d'amides d'acides gras.

Lorsqu'ils se présentent sous forme de sel, les acides gras mono-insaturés peuvent plus particulièrement être des sels cosmétiquement acceptables, c'est-à-dire, des sels inorganiques tels que des sels d'ammonium ou de métaux alcalins (lithium, potassium, sodium) d'alcalino terrreux (magnésium, calcium) ou des sels d'aluminium. En particulier, les acides gras mono-insaturés se présentent sous forme de sel de calcium.

Les acides gras peuvent aussi se présenter sous forme d'esters. Ils peuvent alors être estérifiés avec le glycérol (mono-, di- ou tri-acyl), un alcool comme les alcools méthylique et éthylique, un sucre, un tocophérol, un tocotriénol, un stérol ou encore un alcool gras.

A titre d'acides gras mono-insaturés, on utilise dans la présente invention l'acide pétrosélinique (ou acide delta-6-cis-octadecenoique en C18).

Selon une variante de l'invention, l'acide gras mono-insaturé est utilisé sous une forme isolée, c'est-à-dire après extraction de leur source d'origine.

Selon une autre variante de l'invention, l'acide gras mono-insaturé provient d'extraits végétaux tels que des huiles.

Ainsi l'invention se rapporte notamment à l'utilisation d'une huile riche en l'acide gras mono-insaturé utilisé.

On peut ainsi utiliser des huiles riches en acide pétrosélinique qui ont plus particulièrement choisies parmi les huiles d'ombellifère.

On entend par huile riche en acide pétrosélinique, une huile comprenant au moins 40% d'acide prétrosélinique.

Les ombellifères sont des plantes dont les fleurs sont disposées en ombelles, les espèces particulièrement riches en acide pétrosélénique sont les Umbellifarea-Apiacea et Araliaceae. Les plantes du genre Thapsia sont également des sources d'acide pétrosélénique (Avato et al, Lipids, 2001, 36, 845). Les espèces de préférence utilisées dans l'invention sont le coriandre, le cerfeuil, la carotte, le céleri, le cumin, le carvi, le persil et l'aneth. L'huile d'ombellifère utilisée selon l'invention peut être extraite de la graine de ces ombellifères, par exemple par broyage ou pressage, puis raffinage. L'huile d'ombellifère a une teneur en acide pétrosélinique qui varie selon la graine d'ombellifère d'où elle est extraite. Pour une même ombellifère, la teneur en acide pétrosélinique varie aussi selon le pays d'origine de l'ombellifère et selon l'extraction qui peut être plus ou moins complète.

L'acide pétrosélénique est également un composé abondant (environ 48%) de l'huile de graine de *Gernium sanguneum* (Tsevegsuren et al, Lipids, 2004, 39, 571).

Ainsi, selon un mode de réalisation, l'utilisation objet de la présente invention est telle que l'acide pétrosélinique est utilisé sous forme d'huile d'ombellifère ou de *Gernium sanguneum.*

Selon un mode de réalisation, une composition conforme à la présente invention est telle que la teneur en acide gras mono-insaturée, un des ses sels et/ou un de ses esters est comprise entre 0,0001 et 30 % en poids par rapport au poids total de la composition.

La teneur en acide gras mono-insaturé ou ses dérivés dans les compositions pour la voie orale utilisables selon l'invention sera telle que la dose journalière dudit acide gras mono-insaturé, d'un de ses sels et/ou d'un de ses esters est comprise entre 0,5 et 2500 mg/j, notamment entre 10 et 1200 mg/j, particulièrement entre 5 et 500 mg/j.

Selon un mode particulier de réalisation de l'invention, la teneur en acides gras mono-insaturés dans les compositions utilisables pour la voie orale, est telle que la dose journalière est comprise par exemple entre 5 et 50 mg/j ou entre 150 et 1200 mg/j et notamment entre 150 et 600 mg/j.

Les compositions orales utilisables selon l'invention présentent préférentiellement un support ingérable.

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche.

Bien entendu, les compositions orales selon l'invention peuvent en outre contenir plusieurs autres actifs.

Ces actifs peuvent être choisis parmi les vitamines B1, B3, B5, B6, B8, B12, C, D, E, ou PP, la niacine, les caroténoïddes, les polyphénols et minéraux, les acides aminés, mes catéchines, les oligo-proanthocyanidines, les acides aminés soufrés, les acides gras polyinsaturés oméga 3 et 6 et la gélatine.

Selon une mode de réalisation particulier, ces actifs pourront être choisis parmi ceux permettant de lutter contre le vieillissement du cuir chevelu et améliorer l'aspect de la chevelure, en particulier, on peut utiliser la taurine, la vitamine B6, la cystéine, la vitamine B1 (biotine), la vitamine B12 (bepanthène) et la gélatine.

Les compositions orales peuvent en outre comprendre au moins en probiotique, un prébiotique ou un mélange de probiotique et un mélange de prébiotiques. A titre de microorganismes probiotiques, on peut notamment citer *Lactobacillus johnsonii* ou *Lactobacillus paracaseï.*

Selon un autre de ses aspects, l'invention a pour objet un procédé cosmétique pour améliorer la qualité de la chevelure et de la fibre capillaire comprenant au moins une étape d'ingestion d'une composition orale comprenant, dans un support physiologiquement acceptable, au moins un acide gras mono-insaturé et/ou un de ses esters et/ou un de ses sels, l'acide gras mono-insaturé étant l'acide pétrosélinique.

Le procédé cosmétique de l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

La présente demande décrit également l'utilisation d'au moins un acide gras mono-insaturé, un de ses sels ou un de ses esters pour la fabrication d'une composition dermatologique destinée à traiter et/ou prévenir les pathologies du cuir chevelu et/ou des cheveux, en particulier du cuir chevelu et/ou des cheveux vieillis.

### Exemple 1 - GEL UNIDOSE

| **Principe actif** | **% pds** |
|---|---|
| Huile de pépins de cassis | 10 |
| Huile de coriandre | 10 |
| Lycopene | 10 |
| *Lactobacillus johnsonii* | 10¹⁰ cfu |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On peut prendre une dose de 200 à 400 ml par jour.

### Exemple 2 - CAPSULE

| | **mg/capsule** |
|---|---|
| Huile de fenouil | 300 |
| Huile de pépins de raisin | 200 |
| *Lactobacillus paracaseï* | 10¹⁰ cfu |
| Glycérine | 150 |
| stéarate de magnésium | 0,02 |
| arôme naturel | 0,01 |

On peut prendre une à trois de ces capsules par jour.

### Exemple 3

On adjoint à la formulation de l'exemple 2 un complexe vitaminique comportant 60 mg de vitamine C, 100 µg de vitamine E et 6 mg de β-carotène.

### Exemple 4

On adjoint à la formulation de l'exemple 2, un complexe vitaminique comportant 100 mg de vitamine C, 100 µg de vitamine E et 6 mg de lycopène par capsule.

### Exemple 5

On adjoint à la formulation de l'exemple 2, un complexe vitaminique comportant 100 mg de vitamine B1, B6, B12, 100 µg de taurine et 6 mg de gélatine par capsule.

### Exemple 6 - CAPSULE

| | **mg**/**capsule** |
|---|---|
| Vitamine C | 60 |
| Huile de carotte | 300 |
| Huile de coriandre | 200 |
| Glycérine | 150 |
| stéarate de magnésium | 0,02 |
| arôme naturel | qsp |

On peut prendre une à trois de ces capsules par jour.

### Exemple 7

On adjoint à la formulation de l'exemple 6 un complexe vitaminique comportant 60 mg de vitamine C, 100 µg de vitamine E et 6 mg de β-carotène.

### Exemple 8

On adjoint à la formulation de l'exemple 6 un complexe vitaminique comportant 100 mg de vitamine C, 100 µg de vitamine E et 6 mg de lycopène par capsule.

### Exemple 9

On adjoint à la formulation de l'exemple 6 un complexe vitaminique comportant 100 mg de vitamine B1, B6, B12, 100 µg de taurine et 6 mg de gélatine par capsule.

### Exemple 10: Etude in vitro de l'effet de l'acide pétrosélinique sur la formation des produits de glycation dans des cellules dermiques

Dans le test qui suit, la demanderesse a mis en évidence que l'acide pétrosélinique permet de réduire significativement la formation des produits de glycation dans des cultures de fibroblastes dermiques.

Des fibroblastes dermiques sont mis en culture selon les techniques usuelles, bien connues de l'homme du métier.

La quantification des produits de glycation est déterminée par analyse densitométrique des spots de l'immunodétection des produits de glycation (Méthode Dot Blot), de façon relative, par rapport au témoin (culture de fibroblastes, sans ajout de produit).

On supplémente, dans un cas, la culture de fibroblastes avec de l'aminoguanidine, produit de référence, pour son action sur la diminution significative de la quantité de produit de glycation dans les conditions expérimentales de ce test. Comme indiqué dans le tableau 1 ci-dessous, on détecte la formation de 42% des produits de glycation par rapport au témoin. Ainsi, l'aminoguanidine diminue de 58 % la quantité de produit de glycation par rapport au témoin. Ce résultat permet donc de valider les conditions expérimentales de ce test.

Dans un autre cas, on supplémente la culture de fibroblastes par de l'acide pétrosélinique. Comme indiqué dans le tableau 1 ci-dessous, on détecte la formation de 73% de produits de glycation relativement au témoin, indiquant ainsi une diminution significative (27%) de la formation de produits de glycation par rapport au témoin.

**Tableau 1**

| **Traitement** | **% PG par rapport au témoin** |
|---|---|
| Témoin | 100 |
| Aminoguanidine 1 mg/ml | 42* |
| Acide Pétrosélinique 0,885 mM | 73* |

| | |
|---|---|
| * différence significative par rapport au témoin p<0,01 PG = produits de glycation | |

Ainsi, ce test démontre que l'acide pétrosélinique permet une réduction significative de la formation de produits de glycation dans le derme, conduisant ainsi à lutter contre les altérations du derme dues à l'âge.

Les acides gras mono-insaturés selon l'invention sont donc utiles pour prévenir et/ou à traiter les altérations du derme liées à l'âge, et en particulier les altérations du cuir chevelu et/ou des cheveux apparaissant avec l'âge.

## Revendications

1. Utilisation cosmétique par voie orale d'au moins un acide gras mono-insaturé comportant de 12 à 22 atomes de carbone, un de ses sels ou un de ses esters, pour prévenir et/ou traiter les altérations des cheveux apparaissant avec l'âge non médiées par un processus inflammatoire, l'acide gras mono-insaturé étant l'acide pétrosélinique.

2. Utilisation selon la revendication 1, pour limiter la chute des cheveux et/ou augmenter la densité de la chevelure.

3. Utilisation selon la revendication 1, pour protéger les cheveux vieillis.

4. Utilisation selon la revendication précédente, pour prévenir et/ou limiter la formation de cheveux fins et/ou ternes et/ou cassants et/ou mous.

5. Utilisation selon la revendication 1, pour améliorer la qualité des fibres kératiniques.

6. Utilisation selon la revendication précédente, pour favoriser la pousse de cheveux brillants et/ou épais et/ou vigoureux.

7. Utilisation selon l'une quelconque des revendications précédentes, dans laquelle l'acide gras mono-insaturé est utilisé sous forme isolée ou dans un extrait végétal tel qu'une huile.

8. Utilisation selon l'une quelconque des revendications précédentes, dans laquelle la teneur en acide gras mono-insaturé, un de ses sels et/ou un de ses esters est comprise entre 0,0001 et 30 % en poids par rapport au poids total de la composition.

9. Utilisation selon l'une quelconque des revendications précédentes, dans laquelle la teneur en acide gras mono-insaturé, un de ses sels et/ou un de ses esters dans la composition pour la voie orale est telle que la dose journalière dudit acide gras mono-insaturé, un de ses sels et/ou un de ses esters est comprise entre 0,5 et 2500 mg/j, en particulier entre 10 et 1200 mg/j et particulièrement entre 5 et 500 mg/j.

10. Utilisation selon l'une quelconque des revendications précédentes, dans laquelle l'acide pétrosélinique est utilisé sous forme d'huile d'ombellifère ou de *Gernium sanguneum.*

11. Utilisation selon la revendication10, dans laquelle l'huile d'ombellifère est choisie parmi les huiles de graines de coriandre, cerfeuil, carotte, céleri, cumin, carvi, persil et aneth.

12. Utilisation selon l'une quelconque des revendications précédentes, dans laquelle ledit acide mono-insaturé est utilisé en association avec au moins un actif choisi parmi les vitamines B1, B3, B5, B6, B8, B12, C, D, E, ou PP, la niacine, les caroténoïdes, les polyphénols et minéraux, les acides aminés, les catéchines, les oligo-proanthocyanidines, les acides aminés soufrés, les acides gras polyinsaturés oméga 3 et 6 et la gélatine.

13. Procédé cosmétique pour améliorer la qualité de la chevelure et de la fibre capillaire de cheveux altérés, lesdites altérations des cheveux apparaissant avec l'âge et non médiées par un processus inflammatoire, comprenant l'ingestion d'une composition orale comprenant au moins un acide gras mono-insaturé comportant de 12 à 22 atomes de carbone et/ou un de ses sels et/ou un de ses esters, l'acide gras mono-insaturé étant l'acide pétrosélinique.

## Patentansprüche

1. Orale, kosmetische Verwendung von mindestens einer einfach ungesättigten Fettsäure, die 12 bis 22 Kohlenstoffatome umfasst, einem ihrer Salze oder einem ihrer Ester zur Prävention und/oder Behandlung der mit dem Alter auftretenden Veränderungen der Haare, die nicht durch einen Entzündungsprozess vermittelt werden, wobei die einfach ungesättigte Fettsäure Petroselinsäure ist.

2. Verwendung nach Anspruch 1 zur Begrenzung des Haarausfalls und/oder zur Steigerung der Dichte des Kopfhaares.

3. Verwendung nach Anspruch 1 zum Schutz alternder Haare.

4. Verwendung nach dem vorhergehenden Anspruch zur Verhinderung und/oder Begrenzung der Bildung von feinen und/oder glanzlosen und/oder brüchigen und/oder schwachen Haaren.

5. Verwendung Anspruch 1 zur Verbesserung der Qualität der keratinischen Fasern.

6. Verwendung nach dem vorhergehenden Anspruch zur Förderung des Wachstums von glänzenden und/oder dicken und/oder kräftigen Haaren.

7. Verwendung nach einem der vorhergehenden Ansprüche, wobei die einfach ungesättigte Fettsäure in isolierter Form oder in einem Pflanzenextrakt, wie Öl, verwendet wird.

8. Verwendung nach einem der vorhergehenden Ansprüche, wobei der Gehalt an einfach ungesättigter Fettsäure, einem ihrer Salze und/oder einem ihrer Ester von 0,0001 bis 30 Gew.-% bezogen auf das Gesamtgewicht der Zusammensetzung beträgt.

9. Verwendung nach einem der vorhergehenden Ansprüche, wobei der Gehalt an einfach ungesättigter Fettsäure, einem ihrer Salze und/oder einem ihrer Ester in der Zusammensetzung zur oralen Verabreichung so ist, dass die Tagesdosis der einfach ungesättigten Fettsäure, eines ihrer Salze und/oder einer ihrer Ester 0,5 bis 2500 mg/Tag, insbesondere 10 bis 1200 mg/Tag und besonders 5 bis 500 mg/Tag beträgt.

10. Verwendung nach einem der vorhergehenden Ansprüche, wobei die Petroselinsäure in Form von Doldengewächsöl oder *Geranium sanguineum-*Öl verwendet wird.

11. Verwendung nach Anspruch 10, wobei das Doldengewächsöl aus Koriandersamenöl, Kerbelöl, Karottenöl, Sellerieöl, Kreuzkümmelöl, Kümmelöl, Petersilienöl und Dillöl ausgewählt ist.

12. Verwendung nach einem der vorhergehenden Ansprüche, wobei die einfach ungesättigte Säure in Verbindung mit mindestens einem Wirkstoff verwendet wird, der aus den Vitaminen B1, B3, B5, B6, B8, B12, C, D, E oder PP, Niacin, Carotinoiden, Polyphenolen und Mineralien, Aminosäuren, Katechinen, Oligoproanthocyanidinen, Aminoschwefelsäuren, mehrfach ungesättigten Omega-3- und Omega-6-Fettsäuren und Gelatine ausgewählt ist.

13. Kosmetisches Verfahren zur Verbesserung der Qualität des Kopfhaares und der Haarfaser von veränderten Haaren, wobei die Veränderungen der Haare mit dem Alter auftreten und nicht durch einen Entzündungsprozess vermittelt werden, umfassend die Aufnahme einer oralen Zusammensetzung umfassend mindestens eine einfach ungesättigte Fettsäure, die 12 bis 22 Kohlenstoffatome umfasst und/oder eines ihrer Salze und/oder einen ihrer Ester, wobei die einfach ungesättigte Fettsäure Petroselinsäure ist.

## Claims

1. The cosmetic use via an oral route of at least one mono-unsaturated fatty acid including 12 to 22 carbon atoms, one of its salts or one of its esters, for preventing and/or treating alterations of hair appearing with aging, not mediated by an inflammatory process, the mono-unsaturated fatty acid being petroselic acid.

2. The use according to claim 1, for limiting hair loss and/or increasing density of the hair.

3. The use according to claim 1, for protecting aged hair.

4. The use according to the preceding claim, for preventing and/or limiting the formation of fine and/or dull and/or brittle and/or limp hair.

5. The use according to claim 1, for improving the quality of keratinous fibers.

6. The use according to the preceding claim, for promoting growth of shiny and/or thick and/or strong hair.

7. The use according to any of the preceding claims, wherein the mono-unsaturated fatty acid is used to in an isolated form or in a plant extract such as an oil.

8. The use according to any of the preceding claims, wherein the content of mono-unsaturated fatty acid, one of its salts and/or one of its esters is comprised between 0.0001 and 30% by weight based on the total weight of the composition.

9. The use according to any of the preceding claims, wherein the content of mono-unsaturated fatty acid, one of its salts and/or one of its esters in the composition for the oral route, is such that the daily dose of said mono-unsaturated fatty acid, one of its salts and/or one of its esters is comprised between 0.5 and 2,500 mg/d, in particular between 10 and 1,200 mg/d and particularly between 5 and 500 mg/d.

10. The use according to any of the preceding claims, wherein petroselinic acid is used as an umbelliferous oil or of Gerneum sanguneum.

11. The use according to claim 10, wherein the umbelliferous oil is selected from among oils of coriander, chervil, carrots, celery, cumin, caraway, parsley and dill seeds.

12. The use according to any of the preceding claims, wherein the said mono-unsaturated acid is used in association with at least one active selected from vitamins B1, B3, B5, B6, B8, B12, C, D, E, or PP, niacin,carotenoids, polyphenols and minerals, amino acids, catechins, oligo-proanthocyanidins, sulfur-containing amino acids, omega 3 and 6 polyunsaturated fatty acids and gelatin.

13. A cosmetic method for improving the quality of hair and of the capillary fiber of altered hair, said alterations of hair appearing with age and not mediated by an inflammatory process, comprising the ingestion of an oral composition comprising at least one mono-unsaturated fatty acid including from 12 to 22 carbon atoms and/or one of its salts and/or one of its esters, the mono-unsaturated fatty acid being petroselinic acid.
